# EUROPEAN PATENT SPECIFICATION

(11) **EP 0 758 882 B1**
(45) Date of publication and mention of the grant of the patent: **01.10.2003**
(21) Application number: 95924580.4
(22) Date of filing: 12.06.1995
(51) Int. Cl.: A61K 7/48

(54) **USE OF N-ACETYL-L-CYSTEINE FOR LIGHTENING HYPERPIGMENTED SKIN**
VERWENDUNG VON N-ACETYL-L-CYSTEIN ZUR AUFHELLUNG VON HYPERPIGMENTIERTER HAUT
UTILISATION DE N-ACETYL-L-CYSTEINE POUR ECLAIRCIR UNE PEAU HYPERPIGMENTEE

(30) Priority: 15.06.1994 US 259804
(43) Date of publication of application: 26.02.1997
(73) Proprietor: Textile Research Institute, Inc., Princeton, New Jersey 08542 (US)
(72) Inventor: HILLEBRAND, Greg, George, Fairfield, OH 45014 (US)
(74) Representative: Grünecker, Kinkeldey, Stockmair & Schwanhäusser Anwaltssozietät
(86) International application number: US9507432
(87) International publication number: WO95034280

(56) References cited:
- FR-A- 2 608 425
- FR-A- 2 615 390
- PATENT ABSTRACTS OF JAPAN vol. 12, no. 209 (C-504) (3056) 15 June 1988 & JP,A,63 008 315 (SANSHO SEIYAKU K.K.) 14 January 1988
- PATENT ABSTRACTS OF JAPAN vol. 12, no. 426 (C-542) (3273) 10 November 1988 & JP,A,63 156 708 (KISHIYOUHIN KAGAKU KAIHOU KENKYUSHO K.K.) 29 June 1988
- CHEMICAL ABSTRACTS, vol. 96, no. 2, 11 January 1982, Columbus, Ohio, US; abstract no. 11517g, page 322 ;column L ; & JP,A,81 120 611 (POLA CHEMICAL INDUSTRIES) 22 September 1981
- PATENT ABSTRACTS OF JAPAN vol. 5, no. 200 (C-084) 18 December 1981 & JP,A,56 120 611 (POLA CHEMICAL INC.) 22 September 1981

## Description

The subject invention relates to the field of skin lightening.

Chronic exposure to sunlight results in photoaging of the skin. Photoaging is clinically manifested as multiple adverse effects such as wrinkling, inelasticity and hyperpigmentation (blotchiness or mottling).

Hyperpigmentation is believed to result from changes in the melanocytes present in epidermal cells. Melanocytes, which are located at the base of the epidermis, lose their normal regulation process with aging and produce excess pigment. This excess production leads to the formation of dense perinuclear clumps of melanin in keratinocytes within the epidermis, resulting in areas of hyperpigmentation or "age spots".

Traditional therapy of hyperpigmented skin includes the application of certain skin lightening agents, such as kojic acid, arbutin, hydroquinone or ascorbic acid, which inhibit melanin formation. It is generally known that conditions which result in defective or missing tyrosinase, an enzyme involved in the formation of melanin, lead to a loss of pigmentation, e.g. albinism. See King, R. A. And C. G. Summers, Dermatologic Clinics, Vol. 6 pp. 217-227 (1988).

Tyrosinase is present within the melanosomes in epidermal melanocytes and catalyzes the committed step in the formation of melanin from tyrosine. See Goldsmith, L. A., Physiology, Biochemistry, and Molecular Biology of the Skin, Oxford University Press, pp. 873-903 (N.Y. 1991). Tyrosinase catalyzes the hydroxylation of tyrosine and the oxidation of DOPA to DOPA quinone: 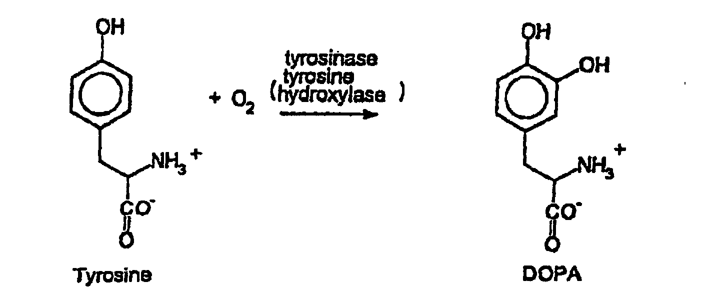 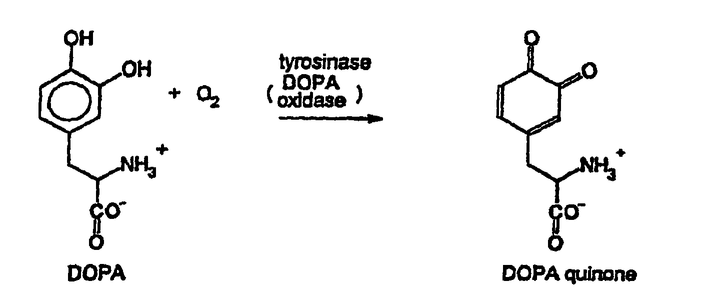 Binding of an inhibitor to the active site of tyrosinase results in decreased melanin formation. See generally Prota, G. Melanins and Melanogenesis, Academic Press, Inc., (San Diego 1992).

Currently, there are tyrosinase inhibitors in the market place to lighten skin, including hydroquinone, kojic acid and arbutin. However, the efficacy of kojic add and arbutin is marginal. Furthermore, hydroquinone has been associated with side effects due to cytotoxicity of the inhibitor's oxidized products.

Additionally, retinoic acid bus been used to normalize the melanocyte population. Retinoic acid also prevents pigment accumulation within the more rapidly dividing and migrating keratinocytes, and enhances the pigment reducing ability of conventional skin lightening agents. However, the topical application of retinoic acid has been associated with skin irritation, thus requiring careful control during use. GB-A-2 265 086 discloses skin whitening agents formulated as patch.

JP-A-63-156 708 discloses a combination of hinokittol and homocysteine, cysteine, ascorbic acid or an ester thereof as skin whitener.

It is the object of the present invention to provide improved skin lightening compositions, providing a safer performance and a better storage behaviour. This object has been solved by the subject matter of claims 1 and 2. Preferred embodiments are described in the subclaims.

It has been unexpectedly found that the use according to this invention achieve skin lightening in hyperpigmented regions in mammalian skin. The subject invention is not limited to any particular mechanism of action, but is believed to operate by the inhibition of tyrosinase, an enzyme crucial for the formation of melanin.

As used herein "hyperpigmented region" means a localized region of high melanin content.

As used herein, "cosmetically- and/or pharmaceutically- acceptable salts" of N-acetyl-L-cysteine include, but are not limited to alkali metal salts, e.g., sodium, lithium, potassium and rubidium salts; alkaline earth metal salts, e.g., magnesium, calcium and strontium salts; non-toxic heavy metal salts, e.g., aluminum and zinc salts; boron salts; silicon salts; ammonium salts; trialkylammonium salts, e.g., trimethylammonium and triethylammonium; and tetralkylonium salts. Preferred cosmetically- and/or pharmaceutically- acceptable salts of the sulfhydryl compound include Na⁺, K⁺, Ca⁺⁺, Mg⁺⁺, Al₂(OH)₅⁺, NH₄⁺, (HOCH₂CH₂)₃NH⁺, (CH₃CH₂)₃NH⁺, (CH₃CH₂)₄N⁺, C₁₂H₂₅(CH₃)₃N⁺ and C₁₂H₂₅(C₅H₄N)₃N⁺ salts. More preferred salts include Na⁺, K⁺, NH₄⁺, and (HOCH₂CH₂)₃NH⁺ salts. Most preferred salts include Na⁺ and NH₄⁺ salts. Suitable salts are described, for example, in U.S. Patent No. 5,296,500, issued to Hillebrand on March 22, 1994, incorporated herein by reference.

As used herein, "topical application" means directly laying on or spreading on outer skin.

As used herein, "cosmetically- and/or pharmaceutically-acceptable-" means that salts, drugs, medicaments, inert ingredients or other materials which the term describes are suitable for use in contact with the tissues of humans and lower animals without undue toxicity, incompatibility, instability, irritation, allergic response, and the like, commensurate with a reasonable benefit/risk ratio.

As used herein, "safe and effective amount' means an amount of compound or composition sufficient to significantly induce a positive modification in the condition to be treated, but low enough to avoid serious side effects (at a reasonable benefit/risk ratio), within the scope of sound medical judgment. As may be applicable to certain uses of the present compositions, the safe and effective amount of the compound or composition will vary with the particular condition being treated, the age and physical condition of the patient being treated, the severity of the condition, the duration of the treatment, the nature of concurrent therapy, the specific compound or composition employed, the particular cosmetically- and/or pharmaceutically-acceptable carrier utilized, and like factors within the knowledge and expertise of an attending physician.

As used herein, "skin lightening" means decreasing melanin in skin, including one or more of lightening of hyperpigmented lesions including age spots, melasma, chloasma, freckles, post inflammatory hyperpigmentation or sun-induced pigmented blemishes.

As used herein, all percentages are by weight unless otherwise specified.

Compositions of this invention preferably comprise from about 0.005% to about 25%, more preferably from about 0.1% to about 15%, still more preferably from about 0.5% to about 10%, yet more preferably from about 1% to about 7%, even more preferably from about 2% to about 5%, most preferably about 2% of N-acetyl-L-cysteine.

### Cosmetically- and/or Pharmaceutically- Acceptable Carrier

The compositions prepared in accordance with the present invention comprise a solid, semi-solid or liquid cosmetically- and/or pharmaceutically- acceptable carrier to enable the sulfhydryl active to be delivered to the desired target at an appropriate concentration. The carrier can itself be inert or it can possess physiological or pharmaceutical benefits of its own. The sulfhydryl active is topically applied to the skin of a subject in need of treatment. Topical application is preferably achieved with compositions in the forms of lotions, solutions, ointments, serums, sprays, tonics, creams, bars, cream rinses, gels, sticks, mousses, pastes and the like.

Topical compositions in accordance with the present invention can be formulated as liquids, for example, as a lotion, mousse or milk. Such liquid compositions may be formulated for use in conjunction with an applicator such as a roll-ball applicator, a pad applicator, or a spray device such as an aerosol can containing propellant, or a container fitted with a pump to dispense the liquid product, or a liquid-impregnated fabric, such as tissue wipe.

Alternatively, the compositions in accordance with the invention can be solid or semi-solid, for example, sticks, serums, creams or gels. Such solid or semi-solid compositions may be formulated for use in conjunction with a suitable applicator or simply a tube, jar or other convenient container.

The selection of a carrier for this purpose presents a wide range of possibilities depending on the required product form of the composition. Suitable vehicles can be classified as described hereinafter.

The term "topical carrier" refers to substances which can act as diluents, dispersants, or solvents for the sulfhydryl active which therefore ensure that it can be applied to and distributed evenly over the selected target at an appropriate concentration. Topical carriers useful in compositions in accordance with the subject invention can include water as a vehicle, and one or more cosmetically- and/or pharmaceutically- acceptable vehicles other than water.

The topical carder is preferably one which can aid and/or enhance penetration into the skin. Carriers useful in topical compositions according to the invention may include penetration enhancers such as liposomes, latex lattices, microspheres, cyclodextrans and various forms of microencapsulation of the sulfhydryl active. A preferred amount of penetration enhancing agent is from about 1% to about 5% of the composition.

Generally, the carrier is either aqueous or organic in nature or an aqueous emulsion, and is capable of having the sulfhydryl active dispersed or dissolved therein. The carder may include cosmetically- and/or pharmaceutically- acceptable emollients, skin penetration enhancers, coloring agents, fragrances, emulsifiers, thickening agents, and/or solvents.

Topical compositions in accordance with the present invention may be formulated as a composition comprising an emollient. Such compositions typically comprise from about 1% to about 50%, preferably from about 5% to about 20% of a topical cosmetically- and/or pharmaceutically- acceptable emollient; and a safe and effective amount of the sulfhydryl active.

As used heron, "emollients" refer to materials used for the prevention or relief of dryness, as well as for the protection of the skin. A wide variety of suitable emollients are known and may be used herein. Such emollients include, but are not limited to, hydrocarbon oils and waxes, silicon oils, triglyceride fats and oils, acetoglyceride esters, ethoxylated glycerides, alkyl esters of fatty acids having 10 to 20 carbon atoms, alkenyl esters of fatty acids having 10 to 20 carbon atoms, fatty acids having 8-22 carbon atoms, fatty alcohols having 8-22 carbon atoms, fatty alcohol ethers, ether-esters, lanolin and derivatives, polyhydric alcohols and their polyether derivatives, wax esters, beeswax derivatives, vegetable waxes, phospholipids, sterols, and amides. SAGARIN, COSMETICS, SCIENCE AND TECHNOLOGY, 2nd Edition, Vol. 1, pp. 32-43 (1972), incorporated herein by reference, contains numerous examples of suitable emollient materials.

Topical compositions in accordance with the subject invention may also be formulated as a cream. Preferably the creams in accordance with the present invention comprise a safe and effective amount of the sulfhydryl active; from about 5% to about 50%, preferably from about 10% to about 25%, of an emollient; and from about 25% to about 95% water. Optionally the cream form contains a suitable emulsifier. When an emulsifier is included, it is in the composition at a level from about 3% to about 50%, preferably from about 5% to about 20%. Emulsifiers may be nonionic, anionic or cationic. Suitable emulsifiers are disclosed in, for example, U.S. Patent No. 3,755,560, issued August 28, 1973, Dickert et aL; U.S. Patent No. 4,421,769, issued December 20, 1983, Dixon et al.; and McCutcheon's Detergents and Emulsifiers, North American Edition, pp. 317-324 (1986); the disclosures of which are incorporated herein by reference. Preferred emulsifiers are anionic or nonionic.

Topical compositions in accordance with the subject invention may also be formulated as a composition comprising a lotion. Preferably the lotions in accordance with the subject invention comprise a safe and effective about of the sulfhydryl active; from about 1% to about 50%, preferably from about 3% to about 15% of an emollient; and from about 45% to about 85%, preferably from about 50% to about 75% water. Optionally, the lotion form may contain a suitable emulsifier, comprising from about 3% to about 50%, preferably from about 10% about 20% of the composition. Examples of suitable emulsifiers are included herein above in the disclosure of cream formulations.

Preferably a solution form in accordance with the present invention comprises a safe and effective amount of the sulfhydryl active, water and a suitable organic solvent. Suitable organic materials useful as the solvent or a part of a solvent system are as follows: propylene glycol, glycerin, polyethylene glycol (M.W. 200-600), polypropylene glycol (M.W. 425.2025), sorbitol esters, 1,2,6-hexanetriol, ethanol, isopropanol, diethyl tartrate butanetliol, and mixtures thereof

Gel compositions in accordance with the present invention can be formulated by simply mixing a suitable thickening agent to the previously described solution compositions. The gel compositions preferably comprise a safe and effective amount of the sulfhydryl active; from about 5% to about 75%, preferably from about 10% to about 50%, of an organic solvent as previously described for solutions; and from about 0.5% to about 20%, preferably from about 1% to about 10% of the thickening agent.

Compositions of solid forms in accordance with the present invention have use as stick-type compositions intended for application to the body. Such compositions preferably comprise a safe and effective amount of the sulfhydryl active, and from about 50% to about 98%, preferably from about 60% to about 90%, of the previously described emollients. Such compositions can further comprise from about 1% to about 20%, preferably from about 5% to about 15%, of a suitable thickening agent, and optionally emulsifiers and water.

The preferred lotions, solutions, sticks, gels, and creams more preferably also contain a preservative, preservative enhancer, zinc and/or a zinc salt as described herein. These agents may be incorporated into the aforementioned formulations in the amounts described herein.

The compositions in accordance with the present invention are formulated to have a pH from about 3 to about 6, most preferably from about 4.5 to about 5.5. Compositions having a pH within the range of about 4.5 to 6 tend to exhibit less skin irritation, less odor, and greater shelf stability relative to corresponding compositions having a pH of greater than about 8.5.

### Other Ingredients

The compositions in accordance with this invention may contain other ingredients, including but not limited to preservatives, preservative enhancers, and actives in addition to the sulfhydryl active. However, certain agents may decrease the activity of the sulfhydryl compound, N-acetyl-L-cysteine, in topical formulations. First, an excessive number of microbial agents may decrease the activity of the sulfhydryl compound, for example by microbial metabolism of the compound. Second, it has been found that formaldehyde may chemically react with the sulfhydryl compound to decrease its activity. Thus, when a composition containing the sulfhydryl compound is formulated with a formaldehyde or a formaldehyde forming preservative or other material, the composition may have decreased activity of the sulfhydryl compound over time relative to the corresponding formulation that does not contain formaldehyde or a compound capable of forming formaldehyde. Therefore, it is desirable to provide compositions containing sulfhydryl compounds that have preservative efficacy and which do not include formaldehyde or formaldehyde forming preservatives or other materials.

The compositions are therefore preferably substantially free of formaldehyde and materials that may form or release formaldehyde when present in the composition, including preservatives that may form or release formaldehyde in the composition. Formaldehyde and materials that may form or release formaldehyde in the composition are alternatively referred to herein as "formaldehyde donor(s)." As used herein, "substantially free of formaldehyde donors" means that there are no detectable formaldehyde donors, preferably no formaldehyde donors. The presence of formaldehyde donors may be indicated by the presence of formaldehyde in the composition by any suitable analytical technique, for example high pressure liquid chromatography. The presence of such donors may be detected initially or evidenced by the generation of formaldehyde over time.

The topical compositions in accordance with the invention preferably comprise one or more preservatives. Preferred preservatives are those which are substantially free of formaldehyde donors. Thus, the preservatives preferred for use herein are those that do not form or release formaldehyde in the composition either in the process of preserving or in an unrelated process. In contrast, formaldehyde forming or releasing preservatives form or release formaldehyde in the composition either in the process of preserving or in an unrelated process.

More preferred preservatives include benzyl alcohol, propylparaben, ethylparaben, butylparaben, methylparaben, benzylparaben, isobutylparaben, phenoxyethanol, ethanol, sorbic acid, benzoic acid, methylchloroisothiazolinone, methylisothiazolinone (a preservative containing a mixture of methylchloroisothiazolinone and methylisothiazolinone being commercially available, for example, from Rohm & Haas as Kathon CG®), methyl dibromoglutaronitrile (commercially available, for example, from Calgon as Tektamer 38®), dehydroacetic acid, o-phenylphenol, sodium bisulfite, dichlorophen, salts of any of the foregoing compounds, and mixtures of any of the foregoing compounds.

Even more preferred preservatives are selected from the group consisting of benzyl alcohol, propylparaben, methylparaben, phenoxyethanol, methylchloroisothiazolinone, methylisothiazolinone, benzoic acid, salts of any of the foregoing preservatives, and mixtures of any of the foregoing compounds,

Still more preferred preservatives are benzyl alcohol, propylparaben, methylparaben, phenoxyethanol and mixtures thereof. Yet even more preferably, the preservative is a mixture of propylparaben and methyl paraben with either or both of benzyl alcohol and phenoxyethanol. In addition to stability of the sulfhydryl compound, these mixtures provide broad preservative efficacy with no or only minimal risk of skin irritation to the user. Most preferably, the preservative is a mixture of benzyl alcohol, propylparaben and methylparaben. In addition to stability of the sulfhydryl compound and broad preservative efficacy, this mixture presents a particularly low risk of skin irritation to the user.

The use of the foregoing preservatives that are substantially free of formaldehyde donors is described in more detail in the copending U.S. Patent Application entitled "Topical Compositions Comprising N-Acetyl-L-Cysteine," filed on June 7, 1995 in the names of Greg. G. Hillebrand and Marcia S. Schnicker, which is incorporated herein by reference in its entirety. The foregoing preservatives are preferably used in the compositions of this invention in the same amounts as described for the compositions of the just referenced patent application.

The compositions in accordance with this invention preferably comprise a safe and effective amount of a preservative enhancer. As used herein, the term "preservative enhancer" means an agent whose purpose is to enhance the activity of the preservative. As will be understood by the artisan having ordinary skill, the preservative enhancer does not itself typically provide sufficient efficacy; it tends to increase the efficacy of the preservative. Enhancement of the preservative efficacy may involve chelation. Preferred preservative enhancers useful in the present invention include ethylenediaminetetraacetic acid (EDTA), butylene glycol, propylene glycol, ethanol, and mixtures thereof. Where the preservative includes a paraben, e.g., methyl or propyl paraben, EDTA is the preferred preservative enhancer. The use of such enhancers is described in more detail in the above-referenced and incorporated copending U.S. Patent Application entitled "Topical Compositions Comprising N-Acetyl-L-Cysteioe," filed on June 7, 1995 in the names of Greg. G. Hillebrand and Marcia S. Schnicker. The preservative enhancers are preferably used in the compositions in accordance with this invention in the same amounts as described for the compositions of the just referenced patent application.

The compositions in accordance with the invention preferably contain zinc or a zinc salt which may complex with the sulfhydryl compound. Without being bound by theory, the zinc most likely removes odor by complexing with malodorous H₂S which may be formed in trace amounts as the sulfhydryl compound decomposes. The zinc may additionally or alternatively increase the stability of the sulfhydryl compound. The use of zinc salts in a manner which is suitable for the present invention is further described in U.S. Patent No. 5,296,500. Hillebrand, issued on March 22, 1994, which is incorporated herein by reference.

The compositions in accordance with the subject invention may optionally comprise other actives capable of functioning in different ways to enhance the benefits of the active (thus, the other actives should not significantly reduce the activity of the sulfhydryl compound).. Examples of such substances include, but are not limited to sunscreens, sunblocks, anti-inflammatory agents, antioxidants/radical scavengers, chelators, and retinoids.

### A. Sunscreens and Sunblocks

Regulation of skin darkening resulting from exposure to ultraviolet light can be achieved by using combinations of the active skin lightening agents together with sunscreens or sunblocks. Useful sunblocks include, for example, zine oxide and titanium dioxide.

Ultraviolet light is a predominant cause of skin darkening. Thus, for purposes of skin lightening, the combination of a skin lightening agent with a UVA and/or UVB sunscreen is desirable.

A wide variety of conventional sunscreening agents are suitable for use in combination with the skin lightening agent. Segarin, et al., at Chapter VIII, pages 189 et seq., of Cosmetics Science and Technology, disclose numerous suitable agents. Specific suitable sunscreening agents include, for example: p-aminobenzoic acid, its salts and its derivatives (ethyl, isobutyl, glyceryl esters; p-dimethylaminobenzoic acid); anthranilates (i.e., o-aminobenzoates; methyl, menthyl, phenyl, benzyl, phenylethyl, linalyl, terpinyl, and cyclohexenyl esters); salicylates (amyl, phenyl, benzyl, menthyl, glyceryl, and dipropyleneglycol esters); Cinnamic acid derivatives (menthyl and benzyl esters, a-phenyl cinnamonitrile; butyl cinnamoyl pyruvate); dihydroxycinnamic acid derivatives (umbelliferone, methylumbelliferone, methylacetoumbelliferone); trihydroxycinnamic acid derivatives (esculetin, methylesculetin, daphnetin, and the glucosides, esculin and daphnin); hydrocarbons (diphenylbutadiene, stilbene); dibenzalacetone and benzalacetophenone; naphtholsulfonates (sodium salts of 2-naphthol-3,6-disulfonic and of 2-naphthol-6,8-disulfonic acids); dihydroxynaphthoic acid and its salts; o- and p-Hydroxybiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives (8-hydroxyquinoline salts, 2-phenylquinoline); hydroxy- or methoxy-substituted benzophenones; uric and vilouric acids; tannic acid and its derivatives (e.g., hexaethylether); (butyl carbotol) (6-propyl piperonyl) ether; hydroquinone; benzophenones (oxybenzene, sulisobenzone, dioxybenzone, benzoresorcinol, 2,2',4,4'-tetra-hydroxybenzophenone, 2,2'dihydroxy-4,4'-dimethoxybenzophenone, octabenzone; 4-isopropyldibenzoylmethane; butylmethoxydibenzoylmethane; etocrylene; and 4-isopropyldibenzoylmethane.

Of these, 2-ethylhexyl-p-methoxycinnamate, 4,4'-t-butylmethoxydibenzoylmethane, 2-hydroxy-4-methoxybenzophenone, octyldimethyl-p-aminobenzoic acid, digalloyltrioleate, 2,2-dihydroxy-4-methoxybenzophenone, ethyl-4-(bis(hydroxypropyl)) aminobenzoate, 2-ethylhexyl-2-cyano-3,3-diphenylacrylate, 2-ethylhexylsalicylate, glyceryl-p-aminobenzoate, 3,3,5-trimethylcyclohexylsalicylate, methylanthranilate, p-dimethylaminobenzoic acid or aminobenzoate, 2-ethylhexyl-p-dimethylaminobenzoate, 2-phenylbenzimidazole-5-sulfonic acid, 2-(p-dimethylaminophenyl)-5-sulfonicbenzoxazoic acid and mixtures of these compounds, are preferred.

More preferred sunscreens useful in the compositions useful in the subject invention are 2-ethylhexyl-p-methoxycinnamate, butylmethoxydibenzoylmethane, 2-hydroxy-4-methoxybenzophenone, octyldimethyl-p-aminobenzoic acid and mixtures thereof.

Also particularly useful in the compositions are sunscreens such as those disclosed in U.S. Patent No. 4,937,370 issued to Sabatelli on June 26, 1990, and U.S. Patent No. 4,999,186 issued to Sabatelli & Spirnak on March 12, 1991, both of which are incorporated herein by reference. The sunscreening agents disclosed therein have, in a single molecule, two distinct chromophore moieties which exhibit different ultra-violet radiation absorption spectra. One of the chromophore moieties absorbs predominantly in the UVB radiation range and the other absorbs strongly in the UVA radiation range.

Preferred members of this class of sunscreening agents are 4-N,N-(2-ethylhexyl)methylaminobenzoic acid ester of 2,4-dihydroxybenzophenone; N,N-di-(2-ethylhexyl)-4-aminobenzoic acid ester with 4-hydroxydibenzoylmethane; 4-N,N-(2-ethylhexyl) methylaminobenzoic acid ester with 4-hydroxydibenzoylmethane; 4-N,N-(2-ethylhexyl)methylaminobenzoic add ester of 2-hydroxy-4-(2-hydroxyethoxy)benzophenone; 4-N,N-(2-ethylhexyl)methylaminobenzoic acid ester of 4-(2-hydroxyethoxy)dibenzoylmethane; N,N-di-(2-ethylhexyl)-4-aminobenzoic acid ester of 2-hydroxy-4-(2-hydroxyethoxy)benzophenone; and N,N-di-(2-ethylhexyl)-4-aminobenzoic acid ester of 4-(2-hydroxyethoxy)dibenzoylmethane and mixtures thereof.

A safe and effective amount of sunscreen may be used in the compositions useful in the subject invention. The sunscreening agent must be compatible with the skin lightening agent. The composition preferably comprises from about 1% to about 20%, more preferably from about 2% to about 10%, of a sunscreening agent. Exact amounts will vary depending upon the sunscreen chosen and the desired Sun Protection Factor (SPF).

An agent may also be added to any of the compositions useful in the subject invention to improve the skin substantivity of those compositions, particularly to enhance their resistance to being washed off by water, or rubbed off. A preferred agent which will provide this benefit is a copolymer of ethylene and acrylic acid. Compositions comprising this copolymer are disclosed in U.S. Patent 4,663,157, Brock, issued May 5, 1987, which is incorporated herein by reference.

### B. Anti-Inflammatory Agents

In a preferred skin lightening composition useful in the subject invention, an anti-inflammatory agent is included as an active along with the skin lightening agent. The inclusion of an anti-inflammatory agent enhances the skin lightening benefits of the compositions. The anti-inflammatory agent protects strongly in the UVA radiation range (though it also provides some UVB protection as well). The topical use of anti-inflammatory agents reduces darkening of the skin resulting from chronic exposure to UV radiation. (See U.S. Patent 4,847,071. Bissett, Bush, and Chatterjee, issued July 11, 1989, incorporated herein by reference; and U.S. Patent 4,847,069, Bissett and Chatterjee, issued July 11, 1989, incorporated herein by reference.)

A safe and effective amount of an anti-inflammatory agent may be added to the compositions useful in the subject invention, preferably from about 0.1% to about 10%, more preferably from about 0.5% to about 5%, of the composition. The exact amount of anti-inflammatory agent to be used in the compositions will depend on the particular anti-inflammatory agent utilized since such agents vary widely in potency.

Steroidal anti-inflammatory agents, including but not limited to, corticosteroids such as hydrocortisone, hydroxyltriamcinalone, alpha-methyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flurnethasone pivalate, fluosinolone acetonide, fluocinonide, flucortine butylester, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone acetonide, medrysone, amcinafel, amcinafide, betamethasone and the balance of its esters, chloroprednisone, chlorprednisone acetate, clocortelone, clescinolone, dichlorisone, difluprednate, flucloronide, flunisolide, fluoromethalone, fluperolone, fluprednisclone, hydrocortisone valerate, hydrocortisone cyclopentylpropionate, hydrocortamate, meprednisone, paramethasone, prednisolone, prednisone, beclomethasone di-propionate, tri-amcinolone, and mixtures thereof may be used. The preferred steroidal anti-inflammatory for use is hydro-cortisone.

A second class of anti-inflammatory agents which is useful in the compositions includes the non-steroidal anti-inflammatory agents. The variety of compounds encompassed by this group are well-known to those skilled in the art. For detailed disclosure of the chemical structure, synthesis, side effects, etc., of non-steroidal anti-inflammatory agents, reference may be had to standard texts, including Anti-inflammatory and Anti-Rheumatic Drugs, K D. Rainsford, Vol. I-III, CRC Press, Boca Raton, (1985), and Anti-inflammatory Agents, Chemistry and Pharmacology, 1, R. A. Scherrer, et al., Academic Press, New York (1974).

Specific non-steroidal antiinflanunatory agents useful in the composition invention include, but are not limited to:
1) the oxicams, such as piroxicam, isoxicam, tenoxicam, sudoxicam, and CP-14,304;
2) the salicylates, such as aspirin, disalcid, benorylate, trilisate, safapryn, solprin, diflunisal, and fendosal;
3) the acetic acid derivatives, such as diclofenac, fenclofenac, indomethacin, sulindac, tolmetin, isoxepac, furofenac, tiopinac, zidometacin, acematacin, fentiazac, zomepiract, clidanac, oxepinac, and felbinac;
4) the fenamates, such as mefenamic, meclofenamic, flufenamic, niflumic, and tolfenamic acids;
5) the propionic acid derivatives, such as ibuprofen, naproxen, benoxaprofen, flurbiprofen, ketoprofen, fenoprofen, fenbufen, indoprofen, pirprofen, carprofen, oxaprozin, pranoprofen, miroprofen, tioxaprofen, suprofen, alminoprofen, and tiaprofenic; and
6) the pyrazoles, such as phenybutazone, oxyphenbutazone, feprazone, azapropazone, and trimethazone.

Mixtures of these non-steroidal anti-inflammatory agents may also be employed, as well as the cosmetically- and/or pharmaceutically- acceptable salts and esters of these agents. For example, etofenamate, a flufenamic acid derivative, is particularly useful for topical application. Of the non-steroidal anti-inflammatory agents, ibuprofen, naproxen, flufenamic acid, mefenamic acid, meclofenamic acid, piroxicam and felbinac are preferred; ibuprofen, naproxen, and flufenamic acid are most preferred.

Another class of anti-inflammatory agents which are useful in the compositions are the anti-inflammatory agents disclosed in U.S. Patent No. 4,708,966, Loomans et al., issued November 24,1987. This patent discloses a class of non-steroidal anti-inflammatory compounds which comprise specifically substituted phenyl compounds, especially substituted 2,6-di- tertbutyl phenol derivatives. For example, compounds selected from 4-(4'-pentyn-3'-one)-2,6-di-t-butylphenol; 4-(5'-hexynoyl)-2,6-di-t-butylphenol; 4-((S)-(-)-3'-methyl-5'-hexynoyl)-2,6-di-t-butylphenol; 4-((R)-(+)-3'-methyl-5'-hexynoyl)-2,6-di-t-butylphenol; and 4-(3',3'-dimethoxypropionyl)-2,6-di-t-butylphenol are useful in methods of the subject invention; 4-(5'-hexynoyl)-2,6-d-t-butylphenol is most preferred.

Yet another class of anti-inflammatory agents which are useful in the compositions are those disclosed in U.S. Patent No. 4,912,248, Mueller, issued March 27, 1990. This patent discloses compounds and diastereomeric mixtures of specific 2-naphthyl- containing ester compounds, especially naproxen ester and naproxol ester compounds, having two or more chiral centers. For example, compounds selected from (S)-naproxen-(S)-2-butyl ester, (S)-naproxen-(R)-2-butylester, (S)-naproxol-(R)-2-methyl butyratc, (S)-naproxol-(S)-2-methyl butyrate, diasteromeric mixtures of (S)-naproxen-(S)-2-butyl ester and (S)-naproxen- (R)-2-butyl ester, and diasteromeric mixtures of (S)-naproxol- (R)-2-methyl butyrate and (S)-naproxol-(S)-2-methyl butyrate are useful in the subject invention.

Finally, so-called "natural" anti-inflammatory agents are useful in methods of the subject invention. For example, candelilla wax, alpha bisabolol, aloe vera, Manjistha (extracted from plants in the genus Rubia, particularly Rubia Cordifolia), and Guggal (extracted from plants in the genus Commiphora, particularly Commiphora Mukul), may be used.

Another preferred composition useful in the subject invention comprises a skin lightening agent, a sunscreen, and an anti-inflammatory agent together for skin lightening in the amounts disclosed for each individually hereinabove.

### C. Anti-Oxidants/Radical Scavengers

In a preferred skin lightening composition useful in the subject invention, an anti-oxidant/radical scavenger is included as an active along with the skin lightening agent. The inclusion of an anti-oxidant/radical scavenger increases the skin lightening benefits of the composition.

A safe and effective amount of an anti-oxidant/radical scavenger may be added to the compositions useful in the subject invention, preferably from about 0.1% to about 10%, more preferably from about 1% to about 5%, of the composition.

Anti-oxidants/radical scavengers such as ascorbic acid (vitamin C) and its salts, tocopherol (vitamin E), tocopherol sorbate, other esters of tocopherol, butylated hydroxy benzoic acids and their salts, 6-hydroxy-2,5,7,8-tetramethylchroman-2-carboxylic acid (commercially available under the tradename Trolox®), gallic acid and its alkyl esters, especially propyl gallate, uric acid and its salts and alkyl esters, sorbic acid and its salts, the ascorbyl esters of fatty acids, amines (e.g., N,N-diethylhydroxylamine, amino-guanidine), sulfhydryl compounds (e.g., glutathione), and dihydroxy fumaric acid and its salts may be used.

In a preferred composition useful in the subject invention, compositions comprise one, any two, or all three of a sunscreening agent, anti-inflammatory agent, and/or an anti-oxidant/radical scavenging agent included as actives along with the skin lightening agent. The inclusion of two or all three of these agents with the skin lightening agent increases the skin lightening benefits of the composition.

### D. Chelators

In a preferred composition useful in the subject invention, a chelating agent is included as an active along with the skin lightening agent. As used herein, "chelating agent" means an active agent capable of removing a metal ion from a system by forming a complex so that the metal ion cannot readily participate in or catalyze chemical reactions. The inclusion of a chelating agent increases the skin lightening benefits of the composition.

A safe and effective amount of a chelating agent may be added to the compositions useful in the subject invention, preferably from about 0.1% to about 10%, more preferably from about 1% to about 5%, of the composition. Chelators useful in compositions are disclosed in U.S. Patent Application Serial No. 619,805, Bissett, Bush & Chatterjee, filed November 27, 1990 (which is a continuation of U.S. Patent Application Serial No. 251,910, filed October 4, 1988); U.S. Patent Application Serial No. 514,892, Bush & Bissett, filed April 26, 1990; and U.S. Patent Application Serial No. 657,847, Bush, Bissett & Chatterjee, filed February 25, 1991; all incorporated herein by reference. Preferred chelators useful in compositions of the subject invention are furildioxime and derivatives thereof.

In a preferred composition useful in the subject invention, compositions comprise one, any two, any three, or all four of a sunscreening agent, anti-inflammatory agent, anti-oxidant/radical scavenging agent, and/or chelating agent included as actives along with the skin lightening agent. The inclusion of two, three, or all four of these agents with the skin lightening agent increases the skin lightening benefits of the composition.

### E. Retinoids

In a preferred composition useful in the subject invention, a retinoid, preferably retinoic acid, is included as an active along with the skin lightening agent. The inclusion of a retinoid increases the skin lightening benefits of the composition. A safe and effective amount of a retinoid may be added to the compositions useful in the subject invention, preferably from about 0.001% to about 2%, more preferably from about 0.01% to about 1% of the composition. As used herein, "retinoid" includes all natural and/or synthetic analogs of Vitamin A or retinol-like compounds which possess the biological activity of Vitamin A in the skin as well as the geometric isomers and stereo isomers of these compounds, such as all-trans retinoic acid and 13-cis-retinoic acid.

In a preferred composition useful in the subject invention, compositions comprise one, any two, any three, any four, and/or all five of a sunscreening agent, anti-inflammatory agent, anti-oxidant/radical scavenging agent, chelating agent, and/or a retinoid included as actives along with the skin lightening agent. The inclusion of two, three, four, or all five of these agents with the skin lightening agent increases the skin lightening benefits of the composition.

The compositions of the present invention are generally prepared by conventional methods such as are known in the art of making topical compositions. Such methods typically involve mixing of the ingredients to a relatively uniform state, with or without heating, cooling, application of vacuum, and the like.

For optimum stability of the sulfhydryl compound, the compositions of this invention should be manufactured, packaged and stored in a manner which avoids simple air oxidation of the sulfhydryl compound, which is well known in the art. Thus, exposure of the compositions to air during manufacture, packaging and storage should be minimized.

### Methods for Lightening Skin in Mammals

A preferred method of applying the subject compositions involves multiple topical application to the face, arm, legs and other areas where hyperpigmented regions are likely to exist. The amount of the composition and the frequency of application can vary widely, depending on the area in questions, the desired effect and/or personal needs, but it is suggested as an example that topical application preferably range from about five times daily, to once every other day, more preferably from about three times daily to once daily, and most preferably about twice daily. The composition for topical application will preferably contain from about 0.001 to about 50 mg of the active per cm² skin receiving the topical composition, more preferably from about 0.01 to about 30 mg/cm², more preferably still from about 0.05 to about 10 mg/cm², also preferably from about 0.1 to about 2mg/cm². The period of topical application should be as is needed by the individual, and may be over the subject's adult life, for continued regulation of hyperpigmentation.

### Examples

Embodiments of the present invention are illustrated in the following non-limiting examples. All parts, percentages, and ratios used herein are by weight unless otherwise specified.

### Example I

A topical composition is prepared by combining the following components utilizing conventional mixing techniques and the pH is adjusted to about 6.0.

| Component | % by weight |
|---|---|
| Thioglycolic acid | 5.0 |
| Propylene glycol | 45.0 |
| Ethanol | 30.0 |
| Water | 20.0 |

1000 mg of the composition per 100 cm² skin is topically applied to a hyperpigmented region on the face twice per day. As the region fades, application is reduced to once a day.

### Example II

A topical composition is prepared by combining the following components utilizing conventional mixing techniques and the is adjusted to about 4.5.

| Component | % by weight |
|---|---|
| Thioglycerol | 2.0 |
| Propylene glycol | 57.0 |
| Ethanol | 20.0 |
| Water | 10.0 |
| Benzyl alcohol | 4.0 |
| Glycerin | 5.0 |
| Myristyl alcohol | 2.0 |

4000 mg of the composition per 100 cm² skin is topically applied once a day to a hyperpigmented region on the upper arm. As the region fades, application is reduced to once every other day.

### Example III

A topical composition is prepared by combining the following components utilizing conventional mixing techniques and the pH is adjusted to about 3.0.

| Component | % by weight |
|---|---|
| Glutathione | 1.0 |
| Propylene glycol | 30.0 |
| Glycerin | 3.0 |
| Water | 66.0 |

2000 mg of the composition per 100 cm² skin is topically applied twice per day to a hyperpigmented region on the back. As the region fades, application is reduced to once every other day.

### Example IV

A topical composition is prepared by combining the following components utilizing conventional mixing techniques and the pH is adjusted to about 5.0.

| Component | % by weight |
|---|---|
| Cysteine | 0.5 |
| Propylene glycol | 30.0 |
| Propylene glycol laurate | 1.0 |
| Isopropanol | 20.0 |
| Water | 48.5 |

500 mg of the composition per 100 cm² skin is topically applied once per day to a hyperpigmented region on the stomach. As the region fades, application is reduced to once a week.

### Example V

A lotion is prepared by combining the following components utilizing conventional mixing techniques and the pH is adjusted to about 4.0.

| Component | % by weight |
|---|---|
| Thiolactic acid | 5.0 |
| Di-partially hydrogenated tallow | |
| dimethyl ammonium chloride | 4.0 |
| Cetyltrimethyl ammonium chloride | 2.0 |
| DC-200 fluid (12500 csk)* | 1.0 |
| Citric acid | 3.5 |
| Ethylene glycol distearate | 1.5 |
| PEG-3 C₁₂ alkyl amide | 3.0 |
| Water | 80.0 |

| | |
|---|---|
| *Dimethylpolysiloxane available from by Dow Chemical Co. | |

100 mg of the composition per 100 cm² skin is topically applied to freckles on the face once every other day. As the region fades, application is reduced to once a week.

### Examples VI - VIII

Lotions are prepared, containing the following compositions, using conventional mixing techniques and the pH adjusted to about 4.5.

| Component | Example No. | | |
|---|---|---|---|
| | VI | VII | VIII |
| | % by weight | % by weight | % by weight |
| Lipoic acid | 0.1 | 0.5 | 2.0 |
| Hydroxylethyl cellulose | 0.4 | - | 0.4 |
| Absolute ethanol | 15.0 | 15.0 | 15.0 |
| Propane-1,2-diol | - | - | 30.6 |
| Butane-1,3-diol | 33.4 | 33.4 | - |
| Paramethyl benzoate | 0.2 | 0.2 | 0.2 |
| Perfume | 0.5 | 0.5 | 0.5 |
| water | 50.4 | 50.4 | 48.7 |

Use of an amount of any of the above compositions to deposit about 750 mg per 100 cm² of the composition to a hyperpigmented region on the back once a day is appropriate. As fading occurs, application is reduced to once every other day.

### Example X

An oil-in-water cream is prepared by mixing the following components and the pH is adjusted to about 3.5.

| Component | % by weight |
|---|---|
| Oily Phase | |
| Thiosalicylic acid | 5.0 |
| Sorbitan monoleate | 20.0 |
| Quaternium-18-hectonite | 5.0 |
| Liquid paraffin | 60.0 |

| Aqueous Phase | |
|---|---|
| Xanthan gum | 1.0 |
| Preservative | 0.3 |
| Perfume | 0.2 |
| Water | 8.5 |

The cream is prepared by mixing the oily phase and heating to 65°C. The aqueous phase is combined and heated to 70°C. The aqueous phase is added to the oil phase with suitable agitation. Moderate agitation is applied while cooling. About 5 mg of the cream is deposited per 100 cm² on a hyperpigmented region on face once a day. As fading occurs, treatment is reduced to once a week.

### Example XI

Prepare an oil-in-water cream by mixing the following components and adjusting the pH to 4.5. Prepare the cream as described for Example X.

| | Component | % by weight |
|---|---|---|
| Oil Phase | | |
| | Perfume | 0.20 |
| | Cetyl alcohol, NF | 1.00 |
| | Stearyl alcohol, NF | 1.00 |
| | Polyoxyethylene (50:50 - 12/20) cetyl/stearyl | 1.00 |
| | ether | |
| | (50:50) | |
| | Propylene glycol dicaprylate/dicaprate | 3.00 |
| | Glycerol monostearate | 2.00 |
| | Glyceryl monostearate-palmitate | 2.00 |

| Water Phase | | |
|---|---|---|
| | N-acetyl-L-cysteine | 5.25 |
| | Distilled Water | 77.19 |
| | Glycerin | 3.00 |
| | Citric acid | 0.50 |
| | Benzyl alcohol | 0.50 |
| | Propylparaben | 0.1 |
| | Methylparaben, NF | 0.25 |
| | Zinc oxide, USP | 0.26 |
| | Butylene glycol | 1.50 |
| | Sodium hydroxide | 1.12 |
| | disodium EDTA | 0.13 |

About 5 mg of the cream is deposited per 100 cm² on a hyperpigmented region on face once a day. As fading occurs, treatment is reduced to once a week.

The cream exhibits enhanced shelf stability, particularly of the N-acetyl-L-cysteine, relative to a corresponding composition which contains a formaldehyde donor such as a preservative which forms or releases formaldehyde in the composition as part of the preservation or another process. Thus, the cream exhibits enhanced N-acetyl-L-cysteine efficacy, relative to the same corresponding composition.

## Claims

1. Use of a safe and effective amount of N-acetyl-L-cysteine, or a cosmetically- and/or pharmaceutically- acceptable salt thereof, for the manufacture of a composition for lightening hyper-pigmented regions in mammalian skin by topical application to the hyper-pigmented regions, the composition further comprising a safe and effective amount of a topical carrier, and wherein the composition has a pH of from 3 to 6.

2. Non-therapeutic use of a safe and effective amount of N-acetyl-L-cysteine, or a cosmetically-and/or pharmaceutically- acceptable salt thereof, for lightening hyper-pigmented regions in mammalian skin by topical application of a composition comprising N-acetyl-L-cysteine to the hyper-pigmented regions, the composition further comprising a safe and effective amount of a topical carrier, wherein the composition has a pH of from 3 to 6.

3. Use according to Claim 1 or Claim 2 wherein the composition has a pH of, from 4.5 to 5.5.

4. Use, according to any preceding claim, wherein the composition is applied to the hyper-pigmented regions from five times daily to once every other day, more preferably from three times daily to once daily, most preferably twice daily.

5. Use, according to any preceding claim, wherein the N-acetyl-L-cysteine, or salt thereof is applied to the hyper-pigmented region in an amount from 0.001 mg per cm² skin to 50 mg per cm² skin, preferably from 0.01 mg per cm2 skin to 30 mg per cm² skin, most preferably from 0.05 mg per cm² skin to 10 mg per cm² skin.

6. Use, according to any preceding claim, wherein the composition further comprises a safe and effective amount of a sunscreen agent or an anti-oxidant.

## Patentansprüche

1. Verwendung einer sicheren und wirksamen Menge von N-Acetyl-L-cystein oder eines kosmetisch und/oder pharmazeutisch annehmbaren Salzes davon für die Herstellung einer Zusammensetzung zum Aufhellen von hyperpigmentierten Regionen in Säugerhaut durch topische Anwendung auf die hyperpigmentierten Regionen, wobei die Zusammensetzung weiterhin eine sichere und wirksame Menge eines topischen Trägers umfasst und wobei die Zusammensetzung einen pH von 3 bis 6 aufweist.

2. Nicht-therapeutische Verwendung einer sicheren und wirksamen Menge von N-Acetyl-L-cystein oder eines kosmetisch und/oder pharmazeutisch annehmbaren Salzes davon zum Aufhellen von hyperpigmentierten Regionen in Säugerhaut durch topische Anwendung einer Zusammensetzung, die N-Acetyl-L-cystein umfasst, auf die hyperpigmentierten Regionen, wobei die Zusammensetzung weiterhin eine sichere und wirksame Menge eines topischen Trägers umfasst, wobei die Zusammensetzung einen pH von 3 bis 6 aufweist.

3. Verwendung nach Anspruch 1 oder Anspruch 2, wobei die Zusammensetzung einen pH von 4,5 bis 5,5 aufweist.

4. Verwendung nach einem der vorhergehenden Ansprüche, wobei die Zusammensetzung auf die hyperpigmentierten Regionen fünfmal täglich bis einmal jeden zweiten Tag, mehr bevorzugt dreimal täglich bis einmal täglich, am meisten bevorzugt zweimal täglich angewandt wird.

5. Verwendung nach einem der vorhergehenden Ansprüche, wobei das N-Actetyl-Lcystein oder Salz davon auf die hyperpigmentierte Region in einer Menge von 0,001 mg pro cm² Haut bis 50 mg pro cm² Haut, vorzugsweise von 0,01 mg pro cm² Haut bis 30 mg pro cm² Haut, am meisten bevorzugt von 0,05 mg pro cm² Haut bis 10 mg pro cm² Haut angewandt wird.

6. Verwendung nach einem der vorhergehenden Ansprüche, wobei die Zusammensetzung weiterhin eine sichere und wirksame Menge eines Sonnenschutzmittels oder eines Antfoxidationsmittels umfasst.

## Revendications

1. Utilisation d'une quantité efficace et inoffensive de N-acétyl-L-cystéine ou d'un sel cosmétiquement et/ou pharmaceutiquement acceptable de celle-ci, pour la fabrication d'une composition d'éclaircissement de régions hyperpigmentées d'une peau d'un mammifère, par application topique sur les régions hyperpigmentées, la composition comprenant en outre une quantité inoffensive et efficace d'un véhicule topique et dans laquelle la composition a un pH de 3 à 6.

2. Utilisation non-thérapeutique d'une quantité efficace et inoffensive de N-acétyl-L-cystéine ou d'un sel cosmétiquement et/ou pharmaceutiquement acceptable de celle-ci, pour l'éclaircissement de régions hyperpigmentées d'une peau d'un mammifère, par application topique d'une composition comprenant de la N-acétyl-L-cystéine sur les régions hyperpigmentées, la composition comprenant en outre une quantité inoffensive et efficace d'un véhicule topique et dans laquelle la composition a un pH de 3 à 6.

3. Utilisation selon la revendication 1 ou la revendication 2, dans lequel la composition a un pH de 4,5 à 5,5.

4. Utilisation selon l'une quelconque des revendications précédentes, dans lequel la composition est appliquée aux régions hyperpigmentées, de cinq fois par jour à une fois tous les deux jours, de préférence de trois à une fois par jour, et de manière préférée entre toutes deux fois par jour.

5. Utilisation selon l'une quelconque des revendications précédentes, dans lequel la N-acétyl-L-cystéine ou le sel de celle-ci est appliqué à la région hyper-pigmentée en une quantité de 0,001 mg par cm² de peau à 50 mg par cm² de peau, de préférence de 0,01 mg par cm² de peau à 30 mg par cm² de peau, de manière préférée entre toutes de 0,05 mg par cm² de peau à 10 mg par cm² de peau.

6. Utilisation selon l'une quelconque des revendications précédentes, dans lequel la composition comprend en outre une quantité efficace et inoffensive d'un filtre solaire ou d'un anti-oxydant.
